Europäisches Patentamt

European Patent Office

Office européen des brevets

(11) Publication number: **0 065 223**

**B1**

# EUROPEAN PATENT SPECIFICATION

(45) Date of publication of patent specification: **04.09.85**

(51) Int. Cl.⁴: **H 01 S 3/10**

(21) Application number: **82103900.5**

(22) Date of filing: **05.05.82**

(54) Laser control device with interval timer.

(30) Priority: **08.05.81 JP 68206/81**

(43) Date of publication of application:
**24.11.82 Bulletin 82/47**

(45) Publication of the grant of the patent:
**04.09.85 Bulletin 85/36**

(84) Designated Contracting States:
**DE FR GB SE**

(56) References cited:
**DE-A-2 900 390**
**FR-A-2 171 866**
**FR-A-2 344 152**

**PATENTS ABSTRACTS OF JAPAN, vol. 5, no. 44 (E-50) (716), 24th March 1981**

(73) Proprietor: **SUMITOMO ELECTRIC INDUSTRIES LIMITED**
**No. 15, Kitahama 5-chome Higashi-ku Osaka-shi Osaka-fu (JP)**

(72) Inventor: **Takenaka, Shinya c/o Osaka Works of Sumitomo**
**Electric Ind. Ltd. No. 1-3, Shimaya 1-chome Konohana-ku Osaka (JP)**

(74) Representative: **Henkel, Feiler, Hänzel & Partner Möhlstrasse 37 D-8000 München 80 (DE)**

Courier Press, Leamington Spa, England.

## Description

### Background of the invention

The present invention relates to a laser control device designed to achieve accurate control of the total radiation energy of a laser beam emitted from the laser.

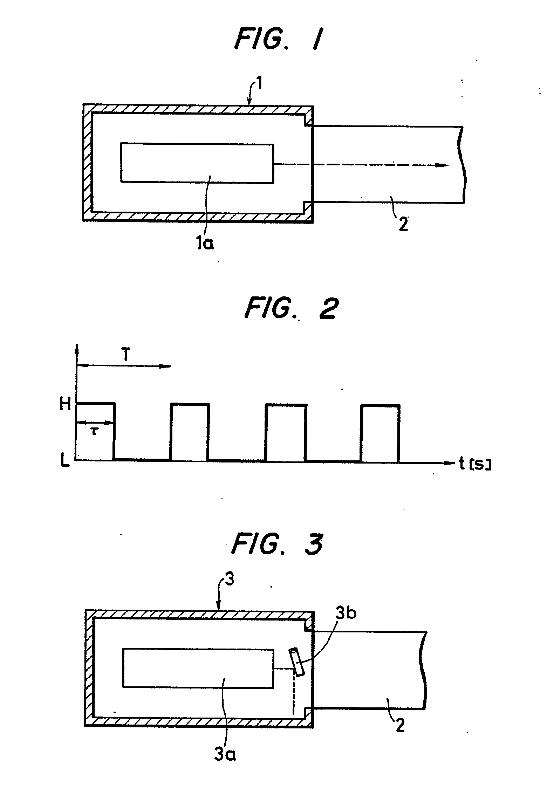

Fig. 1 is a schematic representation of a known laser system 1 which includes a laser emitting tube 1a and a waveguide 2 for directing the laser beam to the target. The laser emitting tube 1a is connected to a laser control device (not shown). Fig. 2 is a characteristic diagram showing a pulse signal transmitted from the laser control device to the laser system. As shown, the laser control device outputs a pulse signal having a pulse width τ to the laser emitting tube 1a at a repetition rate T. The laser system 1 is configured so that it emits a laser beam (shown by the broken line in Fig. 1) when the pulse signal is at a high level H, and does not emit the beam when the pulse signal is at the low level L.

Fig. 3 is a schematic representation of another known laser system 3 which includes a laser emitting tube 3a that generates a continuous laser beam. The laser system 3 also includes a shutter 3b which, upon receiving a pulse signal as shown in Fig. 2, opens or closes depending upon the level thereof. When the pulse signal is at a high level H, the shutter 3b opens to allow the laser beam to be emitted toward the target, and when the pulse signal is at the low level L, the shutter 3b closes so that the laser beam is no longer emitted.

The total radiation energy of the laser beam emitted from the laser system 1 or 3 toward the target is controlled by the following procedure: (1) first, the laser control device is set so that it outputs a pulse signal having a predetermined pulse width τ and repetition rate T; (2) the laser control device is connected to the laser system 1 or 3 and sends the pulse signals to the laser system so that it emits the beam toward the target; and (3) after a predetermined time has passed, the laser control device is manually disconnected from the laser system to halt the emission of the laser beam.

Laser systems of the type illustrated in Figs. 1 and 3 are preferably operated to provide a laser beam having an accurately controlled total radiation energy if they are used in medical applications or for machining purposes. This requirement is particularly great for medical laser systems that can not allow humans to be exposed to any hazard. But it has been difficult to meet such requirements with the conventional technique. The total radiated energy is proportional to the number of pulses included in the pulse signal received by the laser system. The pulse usually has a width of 100 milliseconds (msec) or less whereas the pulse signal being sent from the laser control device to the laser system is manually cut. This means that the control of the pulse signal, which must be effected on the order of msecs to provide a predetermined number of pulses and hence a predetermined level of total radiation energy, is actually effected manually. Accordingly, accurate control of the total radiated energy has been difficult to achieve by the prior art technique.

It should be mentioned that a continuous laser beam and an intermittent laser beam have different effects on the target even if they provide the same total radiation energy. A continuous laser beam having a predetermined total radiation energy can be provided from each of the laser systems 1 and 3 by actuating the systems with a single pulse from the laser control device having a pulse width τ corresponding to the total radiation energy.

In DE—A—29 900 300 a laser control device is shown wherein the number or the total energy of pulses delivered by a laser is monitored by means of a detector interposed in the path of the laser beam, the laser emission being stopped when a given value of the number or of the total energy has been reached. In this known apparatus, only the number or the energy of pulses may be monitored, but there is no means provided for setting the pulse width or the pulse repetition interval. Thus it is not possible to distribute a predetermined total radiation energy over a predetermined time interval.

### Summary of the invention

One object of the present invention is to provide a laser control device that controls accurately the total radiated energy of the laser beam from a laser system designed to emit an intermittent laser beam. This object can be achieved according to the present invention by a control device including a pulse width timer which, upon each setting, sends one pulse having a predetermined pulse width to a laser system for emitting a laser beam when it receives a pulse signal, a pulse cycle timer for cyclically setting the pulse width timer for each preset cycle during the time period from setting to resetting, a pulse repetition interval timer for resetting said pulse cycle timer when a predetermined time has passed following the setting time, and an actuator that simultaneously sets the pulse width timer, pulse cycle timer and pulse repetition interval timer.

### Brief description of the drawings

Fig. 1 is a schematic representation of a pulse-controlled laser system;

Fig. 2 is a diagram showing a pulse train emitted by a control mechanism of the Fig. 1 device;

Fig. 3 is a schematic diagram of a further laser system;

Fig. 4 is a block diagram of a laser control device according to the invention; and

Fig. 5 is a diagram showing the pulse signal output by the device of Fig. 4.

### Detailed description of the preferred embodiments

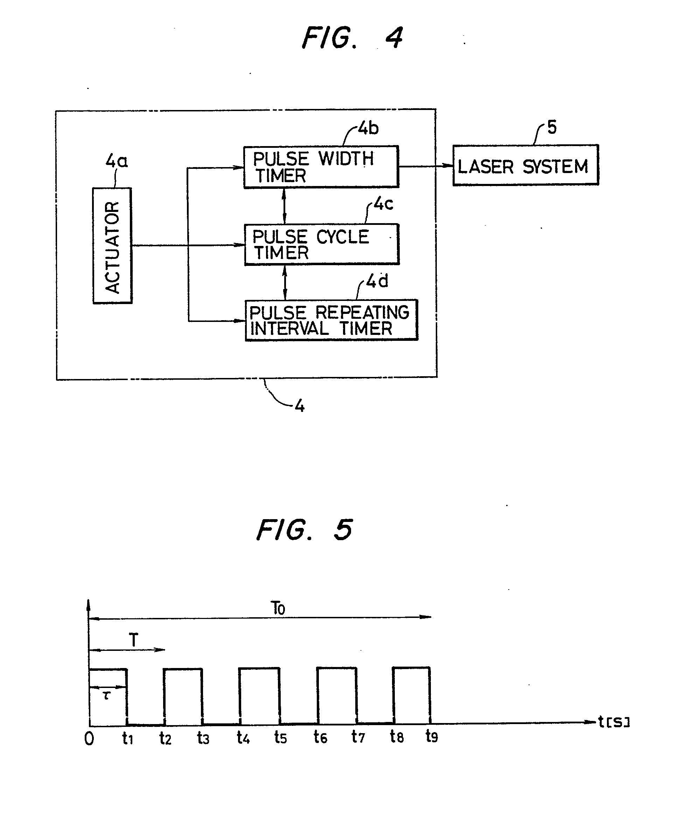

Fig. 4 is a block diagram showing a laser control device according to one preferred embodiment of the present invention, and Fig. 5 is a characteristic diagram showing the pulse signal output by the laser control device of Fig. 4. As shown in Fig. 4, a laser control device with a pulse controlling device generally indicated at 4 includes an actuator 4a, a pulse width timer 4b, a pulse cycle timer 4c and a pulse repetition interval timer 4d. The actuator 4a simultaneously sets the timers 4b, 4c and 4d. The pulse width timer 4b is set by either the actuator 4a or the pulse cycle timer 4c, and each time it is set, it outputs a pulse of width $\tau$ ($t_1[s]$) to a laser system 5, which is identical to either the laser system 1 of Fig. 1 or the laser system 3 of Fig. 3 which in turn emits a laser beam upon receiving each pulse. The pulse cycle timer 4c cyclically sets the pulse width timer 4b at a preset cycle ($t_2[s]$) during the time period defined between setting with the actuator 4a and resetting with the pulse repetition interval timer 4d. Therefore, in the embodiment shown, the pulse width timer 4b is set at each of the time points $t_2$, $t_4$, $t_6$ and $t_8$, and the pulse width timer 4b works in association with the pulse cycle timer 4c to form a pulse signal having a pulse width $\tau=t_1[s]$ and a repetition rate $T=t_2[s]$. The pulse repetition interval timer 4d resets the pulse cycle timer 4c into an OFF mode when a preset time (for example $t_9[s]$) or a pulse repetition interval has passed since the timer 4d was set by the actuator 4a. Therefore, the pulse signal sent from the device of the illustrated embodiment has as many pulses as the number of times the pulse width timer 4b is set by the actuator 4a or the pulse cycle timer 4c within the interval To. More specifically, the pulse signal sent by the control device of the example illustrated has five pulses having a width $\tau=t_1[s]$, and a laser beam having a total radiation energy corresponding to these five pulses is issued from the laser system 5. It is to be understood that each of the timers 4b, 4c and 4d may be constructed of logic circuits such as counters using a basic clock signal. Alternatively, they may be composed of analog circuits.

As described above in conjunction with the preferred embodiment, the laser control device of the present invention has a pulse width timer and pulse cycle timer for producing a pulse signal and also an interval timer that determines the number of pulses to be contained in the pulse signal. Therefore, the device can output to the laser system a pulse signal containing a predetermined number of pulses and thus the laser system is made capable of emitting an intermittent laser beam having a precisely controlled total radiation energy corresponding to the predetermined number of pulses.

**Claims**

1. A laser control device (4) characterized in that it comprises a pulse width timer (4b) for outputting one pulse having a predetermined pulse width upon each setting thereof, a laser system (1; 3) receiving said pulse and emitting a laser beam in response thereto, a pulse cycle timer (4c) for cyclicly setting said pulse width timer (4b) at preset intervals during a time period from the setting thereof to the resetting thereof, a pulse repetition interval timer (4d) for resetting said pulse cycle timer after a predetermined time has passed since the setting thereof, and actuator means (4a) for simultaneously setting said pulse cycle timer, said pulse width timer and said pulse repetition interval timer.

2. A method for operating a laser control device characterized in that initially

— a pulse width timer for outputting one pulse having a predetermined pulse width upon each setting thereof,
— a pulse cycle timer for cyclicly setting said pulse width timer at preset intervals during a time period from the setting thereof to the resetting thereof, and
— a pulse repetition interval timer for resetting said pulse cycle timer after a predetermined time has passed since the setting thereof

are simultaneously set by actuator means, said pulse width timer being subsequently set by said pulse cycle timer, until said pulse cycle timer is reset by said pulse repetition interval timer, said pulses outputted by the pulse width timer being applied to a laser system which emits a laser beam in response thereto.

**Patentansprüche**

1. Laser-Steuergerät (4), dadurch gekennzeichnet, daß es einen Impulsbreitenzeitgeber (4b) zum Abgeben (je) eines Impulses einer vorbestimmten Impulsbreite bei jedem Setzen desselben, ein Laser-System (1; 3), das den Impuls abnimmt und in Abhängigkeit davon einen Laserstrahl emittiert, einen Impulsperiodenzeitgeber (4c) zum zyklischen oder periodischen Setzen des Impulsbreitenzeitgebers (4b) in voreingestellten Intervallen während einer Zeitspanne vom Setzen bis zum Rücksetzen desselben, einen Impulswiederholungsintervall-Zeitgebar (4d) zum Rücksetzen des Impulsperiodenzeitgebers nach Ablauf einer vorbestimmten Zeit seit dem Setzen desselben und eine Betätigungseinrichtung (4a) zum gleichzeitigen Setzen des Impulsperiodenzeitgebers, des Impulsbreitenzeitgebers und des Impulsweiderholungsintervall-Zeitgebers aufweist.

2. Verfahren zum Betreiben eines Laser-Steuergeräts, dadurch gekennzeichnet, daß zunächst

— ein Impulsbreitenzeitgeber zum Abgeben eines Impulses einer vorbestimmten Impulsbreite bei jedem Setzen desselben,
— ein Impulsperiodenzeitgeber zum zyklischen oder periodischen Setzen des Impulsbreitenzeitgebers in voreingestellten Intervallen während einer Zeitspanne vom Setzen bis zum Rücksetzen desselben und

— ein Impulswiederholungsintervall-Zeitgeber zum Rücksetzen des Impulsperiodenzeitgebers nach Ablauf einer vorbestimmten Zeit seit dem Setzen desselben

gleichzeitig durch eine Betätigungseinrichtung gesetzt werden, der Impulsbreitenzeitgeber anschließend durch den Impulsperiodenzeitgeber gesetzt wird, bis der Impulsperiodenzeitgeber durch den Impulswiederholungsintervall-Zeitgeber rückgesetzt wird (und) die vom Impulsbreitenzeitgeber abgegebenen Impulse einem Laser-System, das in Abhängigkeit davon einen Laserstrahl emittiert, eingespeist werden.

**Revendications**

1. Dispositif (4) de commande de laser, caractérisé en ce qu'il comprend une minuterie (4b) de largeur d'impulsion destinée à transmettre une impulsion ayant une largeur prédéterminée après chaque armement, un système laser (1; 3) recevant l'impulsion et émettant un faisceau laser en réponse, une minuterie (4c) de cycles d'impulsions destinée à armer cycliquement la minuterie de largeur d'impulsion (4b) à des intervalles préréglés pendant une période commençant à son armement et se terminant à son réarmement, une minuterie (4d) d'intervalles de répétition d'impulsions destinée à réarmer la minuterie de cycles d'impulsions après l'écoulement d'un temps prédéterminé depuis son armement, et un organe de manoeuvre (4a) destiné à armer simultanément la minuterie de cycles d'impulsions, la minuterie de largeur d'impulsion et la minuterie d'intervalles de répétition d'impulsions.

2. Procédé de manoeuvre d'un dispositif de commande de laser, caractérisé en ce que, initialement,

— une minuterie de largeur d'impulsion destinée à transmettre une impulsion ayant une largeur prédéterminée après chaque armement de la minuterie,
— une minuterie de cycles d'impulsions destinée à armer cycliquement la minuterie de largeur d'impulsion à des intervalles préréglés pendant une période partant de son armement et se terminant à son réarmement, et
— une minuterie d'intervalles de répétition d'impulsions, destinée à réarmer la minuterie de cycles d'impulsions après l'écoulement d'un temps prédéterminé depuis son armement,

sont simultanément armées par un organe de manoeuvre, la minuterie de largeur d'impulsion étant ensuite armée par la minuterie de cycles d'impulsions jusqu'à ce que cette dernière soit réarmée par la minuterie d'intervalles de répétition d'impulsions, les impulsions transmises par la minuterie de largeur d'impulsion étant appliquées à un système laser qui émet un faisceau à la suite de l'impulsion.

0 065 223

*FIG. 1*

*FIG. 2*

*FIG. 3*

1

## FIG. 4

## FIG. 5